# EUROPÄISCHE PATENTSCHRIFT

(11) **EP 1 402 909 B1**
(45) Veröffentlichungstag und Bekanntmachung des Hinweises auf die Patenterteilung: **14.06.2006**
(21) Anmeldenummer: 03019725.5
(22) Anmeldetag: 29.08.2003
(51) Int. Cl.: A61M 1/36, A61M 1/16, A61B 5/087, A61M 25/00

(54) **Vorrichtung, um in einem Zielgebiet eines menschlichen oder tierischen Körpers einen künstlichen isolierten Kreislauf zu etablieren**
Device for establishing an artificial separate circuit within a specific area of a human or animal body
Dispositif pour mettre en place un circuit artificiel dans une région spécifique d' un corps humain ou animal

(30) Priorität: 26.09.2002 DE 10245772
(43) Veröffentlichungstag der Anmeldung: 31.03.2004
(73) Patentinhaber: Maquet Cardiopulmonary AG, 72145 Hirrlingen (DE)
(72) Erfinder: Meiser, Andreas, 44791 Bochum (DE); Mumme, Achim, 44803 Bochum (DE)
(74) Vertreter: Otten, Hajo

(56) Entgegenhaltungen:
- WO-A-01/03755
- WO-A-01/43804
- DE-A- 4 310 378
- FR-A- 2 558 592
- US-A- 5 957 880

## Beschreibung

Die vorliegende Erfindung betrifft eine Vorrichtung mit einer Pumpenanordnung, zumindest einem Venenkatheter und zumindest einem Arterienkatheter, um in einem Zielgebiet eines menschlichen oder tierischen Körpers einen künstlichen Kreislauf zu etablieren und aufrechtzuerhalten, wobei der künstliche Kreislauf von dem Blutkreislauf des Körpers isoliert ist.

Ferner ist ein Verfahren offenbart, bei dem in einem Zielgebiet eines menschlichen oder tierischen Körpers ein künstlicher Kreislauf etabliert und aufrechterhalten wird, der von dem Blutkreislauf des Körpers, der im folgenden auch Körperkreislauf genannt wird, isoliert ist.

Diese sogenannte isolierte Perfusion von Organen oder Körperregionen wird seit längerem angewendet, um in dem Zielgebiet hochwirksame Medikamente zu verabreichen und dabei deren Nebenwirkungen auf den Rest des Organismus zu vermeiden, bzw. Medikamente in solch hohen Konzentrationen einzusetzen, daß bei allgemeiner Anwendung auf den ganzen Körper unzumutbar starke Nebenwirkungen und Unverträglichkeiten auftreten würden.

Unter "Zielgebiet" wird im Rahmen der vorliegenden Erfindung ein von dem restlichen Körper bezüglich des Blutkreislaufes isolierbares Organ oder eine isolierbare Körperregion, wie beispielsweise Extremitäten, also Arm oder Bein, und Becken verstanden. Ein prominentes Beispiel ist die hypertherme isolierte Perfusion mit chemotherapeutischen Substanzen zur Behandlung lokoregionaler Metastasen in Arm oder Bein beim malignen Melanom. Andere Beispiele betreffen die Beckenperfusion bei lokaler Metastasierung eines Rektum-Karzinoms, oder die Bein-Perfusion zur Auflösung sonst therapieresistenter Beinvenenthrombosen. Ein wichtiges Anwendungsgebiet der vorliegenden Erfindung liegt in der zuletzt genannten thrombolytischen Perfusion isolierter Gliedmaßen, vorwiegend der Beine.

Zur Durchführung der isolierten Perfusion wird eine Pumpenanordnung mit zumindest einem Venenkatheter und zumindest einem Arterienkatheter verwendet, die an Arterie und Vene des Ziel gebietes angeschlossen werden, so daß für eine gewisse Zeit ein kleiner künstlicher Kreislauf etabliert und aufrechterhalten werden kann, der vom übrigen Blutkreislauf getrennt ist. Um das Zielgebiet mit Sauerstoff zu versorgen, wird bei längerer Aufrechterhaltung des künstlichen Kreislaufs ferner ein Oxygenator in den künstliche Kreislauf eingeschaltet, durch den ferner der Kohlendioxidspiegel konstantgehalten wird.

Bisher wurden Arterie und Vene des Zielgebietes dafür operativ freigelegt, unter direkter Sicht abgebunden und dann mit dem Arterien- bzw. Venenkatheter verbunden. Mit verbesserter Kathetertechnik ist es heute aber auch möglich, eine isolierte Perfusion nur über Punktion der Gefäße von außen, also ohne Operation, durchzuführen, wobei eine Abtrennung zwischen dem künstlichen Kreislauf und dem Körperkreislauf vorgenommen wird.

Cwikiel et al., "Double-balloon catheter for isolated liver perfusion: an experimental study. *Cardiovasc. Intervent. Radiol*. 2001; 24: 191-3 berichten über eine isolierte Perfusion einer Schweineleber mit vier Kathetern. Die verwendeten Katheter dichteten die Gefäße, in denen sie lagen, mit Hilfe eines an ihnen befestigten Ballons ab.

Die Abtrennung des künstlichen Kreislaufs in dem Zielgebiet von dem Körperkreislauf des restlichen menschlichen oder tierischen Körpers kann somit operativ, mit Hilfe von Kathetern oder aber auch durch Abbinden etc. erfolgen.

Insbesondere dann, wenn bei der isolierten Perfusion hochwirksame, aber toxische Medikamente eingesetzt werden, muß sichergestellt werden, daß der isolierte künstliche Kreislauf isoliert vom restlichen Körperkreislauf arbeitet und eine Leckage der Medikamente in den Blutkreislauf vermieden wird, um die Auswirkungen der verabreichten Medikamente auf den restlichen Körper zu vermeiden.

Untypische Gefäßverläufe bzw. Abgänge, Venengeflechte oder Lymphbahnen können aber häufig weder chirurgisch noch mit der Kathetertechnik vollständig unterbunden werden, so daß es zu einem kontinuierlichen Austausch von Blut zwischen dem künstliche Kreislauf und dem Blutkreislauf des Körpers kommt.

Um diese Gefahr einer Leckage geringzuhalten, wird ein Druckgefälle auf der venösen Seite zwischen den beiden Kreisläufen vermieden. Ist dann der Druck im isolierten künstlichen Kreislauf zu niedrig, so fließt Blut aus dem Körperkreislauf in das therapierte Zielgebiet, so daß das im künstlichen Kreislauf zirkulierende Blutvolumen zunimmt. Nachteiliger ist jedoch der umgekehrte Fall, wenn der Druck im künstlichen Kreislauf zu hoch ist. Dann wird nämlich Blut, das die verabreichten Medikamente enthält, in den Blutkreislauf des restlichen Körpers abgepreßt.

Aus dem Obigen ergibt sich, daß im Zusammenhang mit einer isolierten Perfusion eine zuverlässige Leckagekontrolle extrem wichtig ist. Neben der Sicherstellung eines konstanten Systemvolumens auf der Seite des künstlichen Kreislaufes erfolgt diese Leckagekontrolle bisher mit Hilfe von radioaktiven Substanzen. Sprenger et al., "Quantitative radionuclide leakage control during isolated limb perfusion", *Nuklearmedizin* 1994; 33: 248-53 beschreiben eine Doppelindikatortechnik, bei der mit zwei unterschiedlichen Isotopen markiertes Albumin verwendet wird. Die erste Albumin-Präparation wird dem Blutkreislauf des Körpers zugeführt, um die Lage der Meßsonde über dem Herzen zu kontrollieren. Ein Verrutschen der Meßsonde führt zu einer Veränderung des Signales der Meßsonde. Die zweite Albumin-Präparation wird in einem bestimmten Verhältnis zwischen dem künstlichen Kreislauf und dem Blutkreislauf des Körpers (im folgenden auch Körperkreislauf) aufgeteilt. Solange die Meßsonde über dem Herzen ein konstantes Verhältnis zwischen der ersten und der zweiten Albumin-Präparation erfaßt, ist der künstliche Kreislauf zuverlässig von dem Körperkreislauf isoliert. Ändert sich das Verhältnis, so deutet dies auf eine Leckage hin.

Wenn eine derart erfaßte Leckage nicht durch Änderung der Druckverhältnisse ausgeschaltet werden kann, wird die isolierte Perfusion abgebrochen bzw. die Verabreichung des Medikamentes gestoppt oder gar nicht erst gestartet.

Nachteilig an der Doppelindikatortechnik ist neben der Strahlenbelastung für den Patienten auch der mit dem Umgang mit dem radioaktiven Material verbundene besondere Aufwand, insbesondere die Anforderung an das Personal, bauliche Maßnahmen, die Handhabung der radioaktiven Abfälle etc. Hinzu kommt, daß bei Verwendung von markiertem Albumin eine Leckage über den Lymphstrom nicht erfaßt werden kann.

Ein weiterer Nachteil der Doppelindikatortechnik liegt in den damit verbundenen hohen Kosten wegen des hohen technischen und apparativen Aufwandes, der erforderlichen Personalausstattung sowie der aufgrund gesetzlicher Bestimmungen vorgeschriebenen baulichen Ausstattung.

Nachdem aber gerade die isolierte Perfusion mit der Kathetertechnik - einmal abgesehen von der Leckagekontrolle - auch in kleinen Kliniken problemlos durchgeführt werden kann, besteht ein Bedarf an einem einfacheren Verfahren zur Leckagekontrolle sowie an entsprechenden Vorrichtungen, um das Verfahren durchführen zu können.

Die WO 01/03755 Al offenbart ein Perfusionssystem für Organe, insbesondere für die Leber, mit Bypaß-Kathetern, Perfusions-Kathetern und Verschlußmitteln. Ferner ist ein Pumpensystem vorgesehen mit einer Bypaß-Pumpe und einer Perfusions-Pumpe zur Etablierung eines negativen relativen Drucks am Persfusions-Ausgang. Außerdem weist die Vorrichtung gemäß der WO 01/03755 Al eine Perfusions-Kontrolleinrichtung auf, mit welcher das Pumpensystem dahingehend gesteuert wird, daß mehr Fluid aus dem Organ ausgeführt als zugeführt wird.

Die DE 43 10 378 A1 offenbart ein Perfusionssystem mit einem Pumpsystem, zwei Kathetern und einem Dreiwegehahn, der an einem Katheter angeschlossen ist. Der Dreiwegehahn dient der Einleitung einer Lösung in das Perfusionssystem.

Vor diesem Hintergrund liegt der vorliegenden Erfindung die Aufgabe zugrunde, ein Verfahren sowie eine Vorrichtung zur Leckagekontrolle bei isolierter Perfusion bereitzustellen, bei dem bzw. bei der die obengenannten Nachteile vermieden werden, so daß die Leckagekontrolle preiswert und einfach durchgeführt werden kann.

Bei der eingangs genannten Vorrichtung wird diese Aufgabe erfindungsgemäß gelöst durch erste Mittel zum Einspeisen eines Analysegases in den künstlichen Kreislauf und zweite Mittel zum Überwachen, ob ein Blutaustausch zwischen dem künstlichen Kreislauf und dem Körperkreislauf stattfindet, insbesondere ob das Analysegas aus dem künstlichen Kreislauf in den Körperkreislauf gelangt, wobei die zweiten Mittel zumindest einen Gassensor für das Analysegas aufweisen.

Die der Erfindung zugrunde liegende Aufgabe wird auf diese Weise vollkommen gelöst.

Die Erfinder der vorliegenden Anmeldung haben nämlich erkannt, daß auch die Verwendung eines Analysegases, das in den künstlichen Kreislauf eingespeist wird, eine sichere Leckagekontrolle ermöglicht. Das Analysegas verteilt sich im perfundierten Zielgebiet, das sich entsprechend aufsättigt. Im restlichen Körper ist das Analysegas jedoch nur dann nachzuweisen, wenn der künstliche Kreislauf in den Körperkreislauf "durchbricht" bzw. leckt.

Mit diesem Verfahren läßt sich also im wesentlichen nachweisen, ob Blut aus dem künstlichen Kreislauf in den Körperkreislauf gelangt, was auch der wichtige, zu überprüfende Fall bei der Leckagekontrolle ist. Aber auch wenn Blut aus dem Körperkreislauf in den künstlichen Kreislauf eindringt, läßt sich dies daran erkennen, daß weiteres Analysegas benötigt wird, um auch dieses zusätzliche Blut aufzusättigen.

Vor diesem Hintergrund ist ferner ein Verfahren offenbart, bei dem in einem Zielgebiet eines menschlichen oder tierischen Körpers ein künstlicher Kreislauf etabliert und aufrechterhalten wird, der von dem Körperkreislauf isoliert ist, bei dem in den künstlichen Kreislauf ein Analysegas eingespeist und überwacht wird, ob ein Blutaustausch zwischen dem künstlichen Kreislauf und dem Körperkreislauf erfolgt, insbesondere ob das Analysegas aus dem künstlichen Kreislauf in den Körperkreislauf gelangt.

In der Regel weist die erfindungsgemäße Vorrichtung für die isolierte Perfusion in den ersten Mitteln einen Oxygenator auf, um das Zielgebiet mit Sauerstoff zu versorgen und den Kohlendioxidspiegel konstant zu halten. In diesem Fall ist es technisch besonders einfach, das Analysegas in den künstliche Kreislauf einzuleiten, denn zusätzlich zu der Sauerstoffzufuhr ist an dem Oxygenator noch eine Analysegaszufuhr vorgesehen, in die das Analysegas einleitbar ist, wenn die erfindungsgemäße Vorrichtung zur isolierten Perfusion mit dem menschlichen oder tierischen Körper verbunden ist.

Das Analysegas wird in oder vor dem Oxygenator dem Luftstrom beigemischt und äquilibriert sich mit dem Blut, woraufhin es sich im perfundierten Zielgebiet verteilt. Ist das Zielgebiet aufgesättigt, so erfolgt nur noch eine sehr geringe Nettoaufnahme des Analysegases durch das Blut im Oxygenator, um Verluste per Diffusion durch die Haut oder per Aufnahme durch wenig durchblutete Gewebe wie Fett, Knorpel, Sehnen auszugleichen.

Eine Leckage zwischen dem künstlichen Kreislauf und dem Körperkreislauf läßt sich jetzt mit Hilfe eines Gassensors für das Analysegas nachweisen, so daß die zweiten Mittel zumindest einen Gassensor für das Analysegas aufweisen.

Der Gassensor kann jetzt entweder in einer Atemmaske für die Ausatemluft des Körpers, in dem künstlichen Kreislauf oder in der Abluft des Oxygenators angeordnet sein. Erfindungsgemäß wird also der Gehalt an Analysegas entweder in der Ausatemluft des Körpers oder in dem künstlichen Kreislauf bzw. in der Abluft des Oxygenators gemessen.

Die Erfinder der vorliegenden Anmeldung haben nämlich erkannt, daß es mit einem in einer Atemmaske angeordneten Gassensor für das Analysegas schon bei einer sehr geringen Leckage von Blut aus dem künstlichen Kreislauf in den Körperkreislauf möglich ist, in der Ausatemluft sehr schnell nach dem Eintreten der Leckage das Analysegas nachzuweisen. Der Gassensor kann in diesem Fall in einer Beatmungsmaske angeordnet sein, die bei der isolierten Perfusion in vielen Fällen auch erforderlich ist, um den Körper zu narkotisieren.

Die erfindungsgemäße Vorrichtung umfaßt in diesem Falle also eine Pumpenanordnung, zumindest einen Venenkatheter und zumindest einen Arterienkatheter, einen Oxygenator sowie den Gassensor, der in einer Atemmaske angeordnet ist bzw. in eine bestehende Atemmaske eingebracht wird.

Mit der erfindungsgemäßen Vorrichtung sowie dem erfindungsgemäßen Verfahren in dieser Ausgestaltung ist eine sehr einfache isolierte Perfusion und Leckageüberwachung möglich, chirurgische Eingriffe in den Körper sind zur zuverlässigen Abtrennung des künstlichen Kreislaufes nicht mehr zwingend erforderlich. Durch die Messung des Analysegases in der Ausatemluft können nämlich z.B. die Druckverhältnisse in dem künstlichen Kreislauf so eingestellt werden, daß keine Leckage erfolgt. Zusätzlich kann die Leckageunterbindung durch Abbinden, Ballonkatheter etc. unterstützt werden.

Wenn der Gassensor in dem künstlichen Kreislauf angeordnet ist, so kann anhand seiner Meßsignale zunächst verfolgt werden, wie das Analysegas sich im künstlichen Kreislauf äquilibriert. Sobald dies erfolgt ist, ist nur noch eine sehr geringe weitere Nettoaufnahme des Analysegases erforderlich. Erfolgt jedoch ein Blutaustausch zwischen dem künstlichen Kreislauf und dem Körperkreislauf, so wird eine höhere Menge an Analysegas für die zusätzliche Äquilibrierung benötigt, was mit dem in dem künstlichen Kreislauf angeordneten Gassensor meßtechnisch erfaßt wird.

Eine erfindungsgemäße Vorrichtung umfaßt also in dieser Ausgestaltung die Pumpenanordnung, zumindest einen Arterienkatheter und zumindest einen Venenkatheter, einen Oxygenator sowie einen Gassensor, der auf der Seite des künstlichen Blutkreislaufes mit dem Oxygenator verbunden ist. Mit anderen Worten, der Gassensor befindet sich zwischen dem Oxygenator und einem der verwendeten Katheter.

Bei dieser Vorrichtung sowie dem zugeordneten Verfahren ist von Vorteil, daß zur Etablierung des künstlichen Kreislaufes sowie zu dessen Überwachung lediglich die Katheter mit Venen und Arterien des Zielgebietes verbunden und ggf. entsprechende externe Maßnahmen zur weiteren Abtrennung unternommen werden müssen, die Leckageüberwachung selbst wird dadurch aber bereits sozusagen mit angelegt. Es ist also nicht erforderlich, an der Atemmaske des Patienten zu manipulieren.

Dieses Verfahren ist auch insbesondere dann von Vorteil, wenn die isolierte Perfusion am tierischen Körper vorgenommen wird, beispielsweise bei Haustieren oder Tieren im Zoo, wo die Anlegung einer Atemmaske problematisch sein kann. Auch bei humanen Patienten ergeben sich Vorteile, da eine Beatmung bzw. ein sicheres Abfangen der Ausatemluft bei Spontanatmenden nicht erforderlich ist.

In einem anderen Ausführungsbeispiel ist der Gassensor in der Abluft des Oxygenators angeordnet, wobei vorzugsweise ein weiterer Gassensor in der Analysegaszufuhr angeordnet sein kann.

Eine erfindungsgemäße Vorrichtung umfaßt in diesem Falle also wieder die Pumpenanordnung sowie die Venen- und Arterienkatheter und den Oxygenator, mit dessen Abluft jetzt ein Gassensor verbunden ist. Vorzugsweise befindet sich ein weiterer Gassensor auf der Zufuhrseite für das Analysegas.

Anhand der Meßsignale des Gassensors und ggf. des weiteren Gassensors läßt sich jetzt die Aufnahme von Analysegas durch den künstlichen Kreislauf überwachen. Wenn nach erfolgter Äquilibrierung erneut eine erhöhte Gasaufnahme zu verzeichnen ist, deutet dies auf eine Leckage hin.

Bei dieser Anordnung ist insbesondere von Vorteil, daß der Gassensor in der Abluft des Oxygenators nicht mit Patientenblut in Berührung kommt, so daß er im Gegensatz zu dem Oxygenator und den sonstigen Schläuchen problemlos wiederverwendbar ist.

Um den Komfort und die Sicherheit bei der Verwendung der erfindungsgemäßen Vorrichtung sowie dem Einsatz des erfindungsgemäßes Verfahrens zu steigern, erfolgt die Auswertung der Meßsignale des Gassensors sowie des ggf. vorgesehenen weiteren Gassensors in einer Steuereinheit, die ermittelt, ob eine Leckage vorliegt, und bei Vorliegen einer Leckage den künstlichen Kreislauf abschaltet, zu welchem Zweck sie mit der Pumpenanordnung verbunden ist.

Die Steuereinheit kann natürlich auch noch weitere Aufgaben erfüllen, z.B. kann sie dazu eingerichtet sein, dem behandelnden Personal anzuzeigen, daß der künstliche Kreislauf mit Analysegas gesättigt ist, so daß eine zuverlässige Leckagekontrolle vorliegt und das Medikament in den künstlichen Kreislauf eingespeist werden kann.

Je nach Art des zugeführten Medikamentes kann es auch lediglich erforderlich sein, die Zufuhr des Medikamentes bei Vorliegen einer Leckage zu unterbrechen, so daß der künstliche Kreislauf aufrechterhalten werden kann, wodurch die Versorgung des Zielgebietes sichergestellt ist. Dies ist insbesondere bei einer für längere Zeit vorgesehenen isolierten Perfusion sinnvoll, bei der insbesondere nachts die Steuereinheit eine zuverlässige Sicherheitskontrolle darstellt, wobei auf das Abschalten der Medikamentenzufuhr nicht zwingend sofort reagiert werden muß, da die Versorgung des isolierten Zielgebietes nicht gefährdet ist.

Als Analysegas kommen Gase und sonstige flüchtige Substanzen in Frage, deren Konzentrationen sich in einem Gasgemisch kontinuierlich überwachen lassen und die eine geringe Löslichkeit im Blut aufweisen, so daß sie sehr schnell an- und abfluten und somit eine zeitnahe Erfassung einer Leckage ermöglichen. Sie sollten ferner eine geringe Fettlöslichkeit und damit einen kleinen virtuellen Verteilungsraum besitzen. Geeignet sind damit vor allem Edelgase, besonders bevorzugt ist jedoch die Verwendung des im Krankenhausalltag sowieso häufig vorhandenen Lachgases (N₂O). Lachgas hat den Vorteil, daß es geruchlos, i-nexplosibel, nicht brennbar und nahezu untoxisch ist. Daher wird N₂O in der Herzchirurgie auch heute noch als Narkosegas eingesetzt.

Erste Versuche der Erfinder der vorliegenden Anmeldung haben ergeben, daß die Empfindlichkeit des Gassensors sowie des ggf. vorhandenen weiteren Gassensors für N₂O bei ca. 1 bis 1.000 ppm liegen sollte, damit eine zuverlässige und frühe Detektion einer Leckage sichergestellt ist.

Ein Beispiel für einen Gassensor, der in der Ausatemluft, der Abluft des Oxygenators oder der Zufuhr für Analysegas eingesetzt werden kann, ist der Sensor SENSIT N für das System NARKO-GUARD™ der Firma MSI/Drägerwerk, Lübeck. Bei diesem Gerät liegt die Nachweisgrenze bei 5 ppm N₂O in Luft und die Auflösung bei 0,1 ppm. Es wird zu Messungen am Arbeitsplatz im Zusammenhang mit der Überprüfung von Arbeitsschutzmaßnahmen und als Lecksuchgerät eingesetzt.

Vor diesem Hintergrund wird auch die Verwendung eines Gassensors offenbart, beispielsweise eines N₂O-Sensors, in der erfindungsgemäßen Vorrichtung oder im Zusammenhang mit dem erfindungsgemäßen Verfahren.

Darüber hinaus wird auch die Verwendung eines Oxygenators in der erfindungsgemäßen Vorrichtung oder im Zusammenhang mit dem erfindungsgemäßen verfahren offenbart. Der Oxygenator ist dabei vorzugsweise mit einer Analysegaszufuhr und weiter vorzugsweise mit einem Gassensor oder einem Anschluß dafür an der Abluftseite versehen.

Schließlich betrifft die Erfindung auch einen Kit mit einem Oxygenator, einer Analysegaszufuhr, zumindest einem Venenkatheter und zumindest einem Arterienkatheter und ggf. einem Analysegassensor. Ferner ist die Verwendung dieses Kits in der erfindungsgemäßen Vorrichtung oder in dem dargelegten Verfahren offenbart.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Vorteile ergeben sich aus der folgenden Beschreibung im Zusammenhang mit der beigefügten Zeichnung.

Die einzige Figur zeigt eine schematische Darstellung eines von einem Körperkreislauf isolierten künstlichen Kreislaufes, der über die neue Vorrichtung aufrechterhalten und überwacht wird.

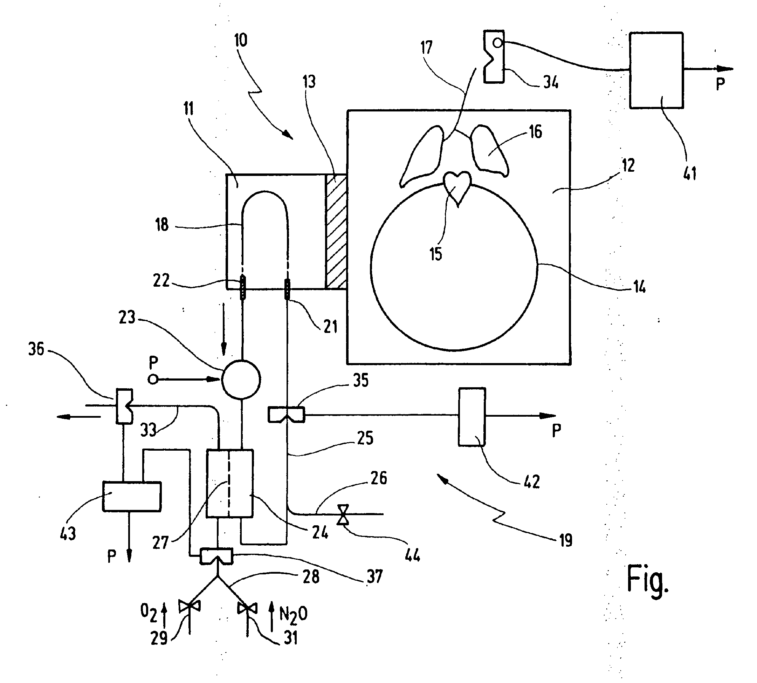

In Fig. 1 ist mit 10 allgemein ein tierischer oder menschlicher Körper gezeigt, bei dem ein mit 11 bezeichnetes Zielgebiet von dem restlichen Körper 12 über eine bei 13 gestrichelt gezeigte Barriere abgetrennt ist. Diese Barriere 13 kann beispielsweise durch chirurgische oder mechanische Maßnahmen erzeugt worden sein.

Schematisch ist in dem Körper 12 noch der Körperkreislauf 14 mit Herz 15 sowie die Lunge 16 und die Luftröhre 17 gezeigt. In dem Zielgebiet 11 ist ein isolierter künstlicher Kreislauf 18 angedeutet, der über eine Vorrichtung 19 etabliert, aufrechterhalten und überwacht wird.

Über die bei 13 gezeigte Barriere ist der künstliche Kreislauf 18 von dem Körperkreislauf 14 abgetrennt, wobei das Zielgebiet 11 ansonsten natürlich mit dem Körper 12 verbunden bleibt. Das Zielgebiet 11 ist beispielsweise ein Bein eines Patienten, das zum Zwecke einer thrombolytischen Behandlung für eine gewisse Zeit mit einem Medikament versorgt werden muß, das aus toxischen oder sonstigen Gründen nicht in den Körperkreislauf 14 gelangen soll. Zu diesem Zweck kann die Barriere 13 dadurch bewirkt werden, daß die Venen und Arterien in dem Zielgebiet 11 von dem Körperkreislauf 14 chirurgisch oder durch mechanische Maßnahmen wie Abdrücken oder Ballonkatheter isoliert werden.

Die Vorrichtung 19 umfaßt einen Arterienkatheter 21 sowie einen Venenkatheter 22, der mit einer Pumpe 23 verbunden ist. An die Pumpe 23 schließt sich ein Oxygenator 24 an, der über einen Schlauch 25 mit dem Arterienkatheter 21 verbunden ist. An dem Schlauch 25 ist über ein Y-Stück eine Medikamenteneinspeisung 26 vorgesehen.

Der Oxygenator 24 beinhaltet eine Gasaustauschmembran 27, die den künstlichen Kreislauf 18 von der Gaszu- und -abfuhr trennt und eine Gas-Äquilibrierung zwischen Gasseite und Blutseite ermöglicht.

Auf der Gasseite ist der Oxygenator 24 mit einem Y-Stück 28 verbunden, das mit einem ersten Anschluß 29 für Sauerstoffzufuhr und einem zweiten Anschluß 31 für die Zufuhr eines Analysegases, hier von Lachgas (N₂O), verbunden ist.

Ferner weist der Oxygenator 24 eine bei 33 angedeutete Abluft auf, aus der der zugeführte Sauerstoff, das zugeführte Lachgas sowie aus dem künstlichen Kreislauf 18 entwichenes Kohlenstoffdioxid entweichen.

Der Oxygenator 24 sowie der als Analysegaszufuhr dienende zweite Anschluß 31 sind Mittel, um das Analysegas N₂O in den künstlichen Kreislauf 18 einzuspeisen. Wenn sich das Analysegas N₂O in dem künstlichen Kreislauf 18 äquilibriert hat, erfolgt nur noch eine sehr geringe Nettoaufnahme von Analysegas, um die Verluste infolge von Diffusion über die Haut oder Aufnahme in wenig durchblutetem Gewebe auszugleichen, sofern der künstliche Kreislauf 18 zuverlässig von dem Körperkreislauf 14 isoliert ist.

Wenn jedoch eine Leckage vorliegt, also zwischen dem künstlichen Kreislauf 18 und dem Körperkreislauf 14 ein Blutaustausch stattfindet, muß über diese geringen Nettoverluste hinaus zusätzliches Analysegas nachgeführt werden.

Die Vorrichtung 19 weist nun Mittel auf, um zu überwachen, ob eine derartige Leckage vorliegt.

Diese Mittel umfassen einen oder mehrere Gassensoren 34, 35, 36 und 37, die einzeln oder in Kombination vorgesehen sein können. Der Gassensor 34 ist in der Ausatemluft des Körpers 12 angeordnet, was vorzugsweise über eine in der Figur nicht dargestellte Atemmaske erfolgt.

Bei einer Leckage von Analysegas aus dem künstlichen Kreislauf 18 in den Körperkreislauf 14 läßt sich nämlich Analysegas N₂O in der Ausatemluft nachweisen. Der Gassensor 34 gibt also an, ob Analysegas und Blut aus dem künstlichen Kreislauf 18 in den Körperkreislauf 14 gelangt.

Ein anderer Gassensor 35 ist in dem künstlichen Kreislauf 18 angeordnet, nämlich zwischen dem Oxygenator 24 und dem Arterienkatheter 21. Dieser Gassensor 35 überprüft den Gehalt an Analysegas in dem künstlichen Kreislauf 18. Bei einem Blutaustausch zwischen dem künstlichen Kreislauf 18 und dem Körperkreislauf 14 verändert sich der Gehalt an Analysegas über die im äquilibrierten Zustand vorhandene Veränderung hinaus, so daß der Gassensor 35 eine Leckage erkennt.

Der Gassensor 36 ist mit dem Abluftschlauch 33 des Oxygenators 24 verbunden. Bei konstanter Zufuhr von Analysegas N₂O über den zweiten Anschluß 31 gibt der Gassensor 36 ein konstantes Signal ab, wenn das Analysegas N₂O in den künstlichen Kreislauf 18 äquilibriert ist, lediglich die geringe Nettoaufnahme zum Diffusionsausgleich verändert das Signal des Gassensors 36. Bei einer Leckage, wenn also vermehrt Analysegas über den Oxygenator 24 in den künstlichen Kreislauf 18 gelangt, zeigt der Gassensor 36 jedoch ein reduziertes Signal an, woraus auf eine Leckage geschlossen werden kann.

Um Schwankungen in der Zufuhr des Analysegases N₂O zu berücksichtigen, kann ein weiterer Gassensor 37 in der Gaszufuhr zu dem Oxygenator 24 vorgesehen sein. Bei dieser Anordnung gibt das Differenzsignal zwischen den Gassensoren 36 und 37 die Nettoaufnahme von Analysegas in dem künstlichen Kreislauf 18 an und kann ebenfalls zur Überwachung der Leckage verwendet werden.

Die einzelnen Gassensoren 34, 35, 36, 37 sind mit zugeordneten Steuereinheiten 41, 42, 43 verbunden, die ihrerseits mit der Pumpe 23 verbunden sind.

Die Steuereinheiten 41, 42, 43 erfassen den Gehalt an Analysegas N₂O und schalten die Pumpe 23 ab, wenn aus den Signalen der Gassensoren 34, 35, 36, 37 eine Leckage ersichtlich ist. Auf diese Weise wird verhindert, daß bei einer Leckage zugeführtes Medikament über den künstlichen Kreislauf 18 in den Körperkreislauf 14 gelangt.

Die Steuereinheiten 41, 42, 43 können statt mit der Pumpe 23 auch mit einer in der Figur nicht gezeigten Zufuhr für das Medikament verbunden sein, so daß bei einer Leckage der künstliche Kreislauf 18 zwar aufrechterhalten bleibt, die Zufuhr des Medikamentes über die Medikamenteneinspeisung 26 jedoch verhindert wird. Dies kann beispielsweise dadurch erfolgen, daß ein bei 44 angedeutetes Ventil in der Medikamenteneinspeisung 26 geschlossen wird.

Insbesondere die Steuereinheiten 42 und 43 können darüber hinaus auch im Zusammenhang mit der Etablierung des künstlichen Kreislaufes 18 verwendet werden. Wenn nämlich diese Steuereinheiten 42, 43 anzeigen, daß keine oder nur noch eine sehr geringe Veränderung des Gehaltes oder der Aufnahme an Analysegas N₂O vorliegt, so bedeutet dies, daß der künstliche Kreislauf 18 isoliert und das Zielgebiet 11 mit Analysegas N₂O gesättigt ist. Wenn dieser Zustand erreicht ist, ist eine zuverlässige Leckageüberwachung möglich, und das Medikament kann in den künstlichen Kreislauf 18 eingespeist werden.

Schließlich können die Signale der Steuereinheiten 41, 42, 43 auch vor der Einspeisung des Medikamentes dazu herangezogen werden, um die Isolation des künstlichen Kreislaufes 18 zu überprüfen. Sofern sich nämlich eine Leckage abzeichnet, kann dieser beispielsweise durch Änderung des Druckes an der Pumpe 23 oder durch weitere Maßnahmen an der Barriere 13 gegengewirkt werden.

## Patentansprüche

1. Vorrichtung mit einer Pumpenanordnung (23), zumindest einem Venenkatheter (22) und zumindest einem Arterienkatheter (21), um in einem Zielgebiet (11) eines menschlichen oder tierischen Körpers (12) einen künstlichen Kreislauf (18) zu etablieren und aufrechtzuerhalten, wobei der künstliche Kreislauf (18) von dem Blutkreislauf des Körpers (Körperkreislauf 14) isoliert ist,
**gekennzeichnet durch** erste Mittel (24, 31) zum Einspeisen eines Analysegases in den künstlichen Kreislauf (18), und zweite Mittel (34, 35, 36, 37, 41, 42, 43) zum Überwachen, ob ein Blutaustausch zwischen dem künstlichen Kreislauf (18) und dem Körperkreislauf (14) stattfindet, insbesondere ob das Analysegas aus dem künstlichen Kreislauf (18) in den Körperkreislauf (14) gelangt, wobei die zweiten Mittel zumindest einen Gassensor (34, 35, 36, 37) für das Analysegas aufweisen.

2. Vorrichtung nach Anspruch 1, **dadurch gekennzeichnet, daß** die ersten Mittel einen Oxygenator (24) mit einer Analysegaszufuhr (31) aufweisen, in die das Analysegas einleitbar ist.

3. Vorrichtung nach Anspruch 2, **dadurch gekennzeichnet, daß** der Gassensor (34) in einer Atemmaske für die Ausatemluft des Körpers (12) angeordnet ist.

4. Vorrichtung nach Anspruch 2, **dadurch gekennzeichnet, daß** der Gassensor (35) in dem künstlichen Kreislauf (18) angeordnet ist.

5. Vorrichtung nach Anspruch 2, **dadurch gekennzeichnet, daß** der Gassensor (36) in der Abluft (33) des Oxygenators (24) angeordnet ist.

6. Vorrichtung nach Anspruch 5, **dadurch gekennzeichnet, daß** ein weiterer Gassensor (37) vorgesehen ist, der in der Analysegaszufuhr (28) angeordnet ist.

7. Vorrichtung nach einem der Ansprüche 2 bis 6, **dadurch gekennzeichnet, daß** eine Steuereinheit (41, 42, 43) vorgesehen ist, die mit dem zumindest einen Gassensor (34, 35, 36) und der Pumpenanordnung (23) verbunden ist, um bei einem Blutaustausch zwischen dem künstlichen Kreislauf (18) und dem Körperkreislauf (14), insbesondere bei einem Durchbrechen des Analysegases in den Körperkreislauf (14) den künstlichen Kreislauf (18) abzuschalten.

8. Vorrichtung nach Anspruch 7, **dadurch gekennzeichnet, daß** die Steuereinheit (43) mit dem weiteren Gassensor (37) verbunden ist.

9. Vorrichtung nach einem der Ansprüche 1 bis 8, **dadurch gekennzeichnet, daß** das Analysegas Lachgas (N₂O) ist, und der zumindest eine Gassensor (34, 35, 36) sowie der ggf. vorhandene weitere Gassensor (37) ein N₂O-Sensor sind.

10. Vorrichtung nach Anspruch 9, **dadurch gekennzeichnet, daß** der zumindest eine Gassensor (34, 35, 36) sowie der ggf. vorhandene weitere Gassensor (37) eine Empfindlichkeit für N₂O von ca. 1 bis 1.000 ppm aufweist.

11. Kit mit einem Oxygenator (24), einer Analysegaszufuhr (31), zumindest einem Venenkatheter (22), zumindest einem Arterienkatheter (21) und einem Analysegassensor (34, 35, 36).

## Claims

1. A device with a pump arrangement (23), at least one venous catheter (22), and at least one arterial catheter (21), for establishing and maintaining an artificial circulation (18) in a target area (11) of a human or animal body (12), the artificial circulation (18) being isolated from the blood circulation of the body (systemic circulation 14),
**characterized by** first means (24, 31) for feeding an analysis gas into the artificial circulation (18), and second means (34, 35, 36, 37, 41, 42, 43) for monitoring whether a blood exchange takes place between the artificial circulation (18) and the systemic circulation (14), in particular whether the analysis gas passes from the artificial circulation (18) into the systemic circulation (14), wherein the second means comprise at least one gas sensor (34, 35, 36, 37) for the analysis gas.

2. The device according to claim 1, **characterized in that** the first means comprise an oxygenator (24) with an analysis gas delivery line (31), into which the analysis gas can be introduced.

3. The device according to claim 2, **characterized in that** the gas sensor (34) is arranged in a respiratory mask for the air exhaled from the body (12).

4. The device according to claim 2, **characterized in that** the gas sensor (35) is arranged in the artificial circulation (18).

5. The device according to claim 2, **characterized in that** the gas sensor (36) is arranged in the air outlet line (33) of the oxygenator (24).

6. The device according to claim 5, **characterized in that** a further gas sensor (37) is provided which is arranged in the analysis gas delivery line (28).

7. The device according to any of claims 2 through 6, **characterized in that** a control unit (41, 42, 43) is provided which is connected to the at least one gas sensor (34, 35, 36) and to the pump arrangement (23), for switching off the artificial circulation (18) in the event of a blood exchange between the artificial circulation (18) and the systemic circulation (14), in particular in the event of the analysis gas breaking through into the systemic circulation (14).

8. The device according to claim 7, **characterized in that** the control unit (43) is connected to the further gas sensor (37).

9. The device according to any of claims 1 through 8, **characterized in that** the analysis gas is laughing gas (N₂O), and the at least one gas sensor (34, 35, 36) and the optionally present further gas sensor (37) are N₂O sensors.

10. The device according to claim 9, **characterized in that** the at least one gas sensor (34, 35, 36) and the optionally present further gas sensor (37) have an N₂O sensitivity of ca. 1 to 1000 ppm.

11. A kit with an oxygenator (24), an analysis gas delivery line (31), at least one venous catheter (22), at least one arterial catheter (21) and, optionally, an analysis gas sensor (34, 35, 36).

## Revendications

1. Dispositif comprenant un agencement de pompe (23), au moins un cathéter pour veines (22) et au moins un cathéter pour artères (21), afin d'établir et de maintenir un circuit (18) artificiel dans une zone cible (11) d'un corps (12) humain ou animal, le circuit (18) artificiel étant isolé du circuit sanguin du corps (circuit du corps 14),
**caractérisé par** des premiers moyens (24, 31) pour l'injection d'un gaz d'analyse dans le circuit (18) artificiel et des seconds moyens (34, 35, 36, 37, 41, 42, 43) pour vérifier si un échange de sang a lieu entre le circuit (18) artificiel et le circuit du corps (14), en particulier si le gaz d'analyse provenant du circuit (18) artificiel parvient dans le circuit du corps (14), les seconds moyens présentant au moins un capteur de gaz (34, 35, 36, 37) pour le gaz d'analyse.

2. Dispositif selon la revendication 1, **caractérisé en ce que** les premiers moyens présentent un oxygénateur (24) avec une arrivée de gaz d'analyse (31), dans laquelle le gaz d'analyse peut être introduit.

3. Dispositif selon la revendication 2, **caractérisé en ce que** le capteur de gaz (34) est disposé dans un masque respiratoire pour l'air expiré du corps (1 2).

4. Dispositif selon la revendication 2, **caractérisé en ce que** le capteur de gaz (35) est disposé dans le circuit (18) artificiel.

5. Dispositif selon la revendication 2, **caractérisé en ce que** le capteur de gaz (36) est disposé dans l'air évacué (33) de l'oxygénateur (24).

6. Dispositif selon la revendication 5, **caractérisé en ce qu'**il est prévu un autre capteur de gaz (37), qui est disposé dans l'arrivée du gaz d'analyse (28).

7. Dispositif selon l'une quelconque des revendications 2 à 6, **caractérisé en ce qu'**il est prévu une unité de commande (41, 42, 43), qui est reliée à le au moins un capteur de gaz (34, 35, 36) et l'agencement de pompe (23), afin de déconnecter le circuit (18) artificiel lors d'un échange de sang entre le circuit (18) artificiel et le circuit du corps (14), en particulier lors d'une entrée brusque du gaz d'analyse dans le circuit du corps (14).

8. Dispositif selon la revendication 7, **caractérisé en ce que** l'unité de commande (43) est reliée à l'autre capteur de gaz (37).

9. Dispositif selon l'une quelconque des revendications 1 à 8, **caractérisé en ce que** le gaz d'analyse est du gaz hilarant (N₂O) et le au moins un capteur de gaz (34, 35, 36) et l'autre capteur de gaz (37) éventuellement présents sont un capteur à N₂O.

10. Dispositif selon la revendication 9, **caractérisé en ce que** le au moins un capteur de gaz (34, 35, 36) et l'autre capteur de gaz (37) éventuellement présent présentent une sensibilité pour le N₂O d'environ 1 à 1.000 ppm.

11. Kit comprenant un oxygénateur (24), une arrivée de gaz d'analyse (31), au moins un cathéter pour veines (22), au moins un cathéter pour artères (21) et un capteur de gaz d'analyse (34, 35, 36).
